# EUROPEAN PATENT SPECIFICATION

(11) **EP 2 449 950 B1**
(45) Date of publication and mention of the grant of the patent: **12.02.2020**
(21) Application number: 11184808.1
(22) Date of filing: 12.10.2011
(51) Int. Cl.: A61B 1/00, A61B 1/06, A61B 1/07

(54) **ENDOSCOPIC DIAGNOSIS SYSTEM**
Endoskopisches Diagnosesystem
Système de diagnostic endoscopique

(30) Priority: 09.11.2010 JP 2010250562
(43) Date of publication of application: 09.05.2012
(73) Proprietor: Fujifilm Corporation, Minato-ku Tokyo (JP)
(72) Inventor: Saito, Takaaki, Kanagawa (JP)
(74) Representative: Klunker IP Patentanwälte PartG mbB

(56) References cited:
- EP-A2- 2 368 486
- JP-B2- 2 648 494
- US-A- 4 914 512
- US-A- 4 951 133
- US-A- 4 998 973
- US-A1- 2009 137 908
- US-A1- 2009 247 881

## Description

### BACKGROUND OF THE INVENTION

The present invention relates to an endoscopic diagnosis system that calculates information on a blood hemoglobin oxygen saturation level based on an image acquired by an endoscope device and displays an oxygen saturation level distribution as pseudo color image.

In recent years, a number of diagnoses and treatments using electronic endoscopes have been made in the field of medicine. A typical electronic endoscope is equipped with an elongated insertion section that is inserted into a subject's body cavity. The insertion section has therein incorporated an imager such as a CCD at the tip thereof. The electronic endoscope is connected to a light source device, which emits light from the tip of the insertion section to illuminate the inside of a body cavity. With the inside of the body cavity illuminated by light, the subject tissue inside the body cavity is imaged by an imager provided at the tip of the insertion section. Images acquired by imaging undergoes various kinds of processing by a processor connected to the electronic endoscope before being displayed by a monitor. Thus, the electronic endoscope permits real-time observation of images showing the inside of the subject's body cavity and thus enables sure diagnoses.

The light source device uses a white light source such as a xenon lamp capable of emitting white broadband light whose wavelength ranges from a blue region to a red region. Use of white broadband light to illuminate the inside of a body cavity permits observing the whole subject tissue from the acquired images thereof. However, although images acquired by broadband light illumination permit generally observing the whole subject tissue, there are cases where such images fail to enable clear observation of subject tissues such as micro-blood vessels, deep-layer blood vessels, pit patters, and uneven surface profiles formed of recesses and bumps. As is known, such subject tissues may be made clearly observable when illuminated by narrowband light having a wavelength limited to a specific range. As is also known, image data obtained by illumination with narrowband light yields various kinds of information on a subject tissue such as oxygen saturation level in a blood vessel, and the acquired information is converted into an image.

JP 2648494 B, for example, describes acquiring an oxygen saturation level image using narrowband light by separating three narrowband light IR1, IR2, and IR3 each having different wavelengths in near infrared range or three narrowband light G1, G2, and G3 each having different wavelengths in visible light range from broadband light emitted from a xenon lamp using a band-limiting filter to obtain images produced by narrowband light by the frame sequential method. Both combinations contain two narrowband light having a wavelength band in which the degree to which light is absorbed (absorbance) changes according to the blood hemoglobin oxygen saturation level and one narrowband light having a wavelength band in which the absorbance does not change. JP 2648494 B describes selecting two of the three signals corresponding to the three narrowband light having different wavelengths and detecting the differences among them to display an oxygen saturation level image in monochrome or in pseudo colors. Endoscopes of interest are also known from US 4,914,512, EP 2 368 486, US 2009/247881, and US 5,408,998.

### SUMMARY OF THE INVENTION

Generally, the reflectance of light from a body tissue depends on three factors: blood hemoglobin oxygen saturation level; blood vessel depth; and blood amount (blood vessel diameter or blood vessel density). Accordingly, the method described in JP 2648494 B, though capable of displaying an oxygen saturation level distribution as an image, does not consider effects produced by the change in blood vessel depth and blood amount on the oxygen saturation level and is therefore incapable of accurately calculating the oxygen saturation level.

An object of the present invention is to provide an endoscopic diagnosis system capable of accurately calculating the oxygen saturation level by considering effects produced thereon by the blood vessel depth and the blood amount and displaying an oxygen saturation level distribution as pseudo color image (false color or simulated color).

In order to achieve the above-described objects, the present invention provides an endoscopic diagnosis system comprising the features of the independent claims.

The present invention enables acquisition of information on the blood amount and the oxygen saturation level while reducing the effects produced by the blood vessel depth. The present invention is capable of accurately calculating information on the oxygen saturation level from an image signal by considering the blood amount and displaying an oxygen saturation level distribution as pseudo color image (false color or simulated color).

### BRIEF DESCRIPTION OF THE DRAWINGS

Fig. 1 is an external view of an embodiment illustrating a configuration of the endoscopic diagnosis system according to the invention.
Fig. 2 is a block diagram of a first embodiment illustrating an internal configuration of the endoscopic diagnosis system shown in Fig. 1.
Fig. 3 is a front view of the tip portion of the endoscope.
Fig. 4 is a graph illustrating an emission spectrum of a blue laser beam emitted from a blue laser light source and light obtained through wavelength conversion of blue laser beam by a fluorescent body.
Fig. 5 is a graph illustrating spectral transmittances of red, green, and blue filters.
Fig. 6 is a graph illustrating light absorption coefficients of hemoglobin.
Fig. 7 is a graph illustrating a correlation between signal ratios B/G and R/G on the one hand and blood amount and oxygen saturation level on the other hand.
Fig. 8 is a block diagram of a second embodiment illustrating an internal configuration of the endoscopic diagnosis system shown in Fig. 1.
Fig. 9 is a block diagram of a third embodiment illustrating an internal configuration of the endoscopic diagnosis system shown in Fig. 1.
Fig. 10 is a block diagram of a fourth embodiment illustrating an internal configuration of the endoscopic diagnosis system shown in Fig. 1.

### DETAILED DESCRIPTION OF THE INVENTION

The endoscopic diagnosis system according to the present invention will be described in detail based on the preferred embodiments illustrated in the attached drawings.

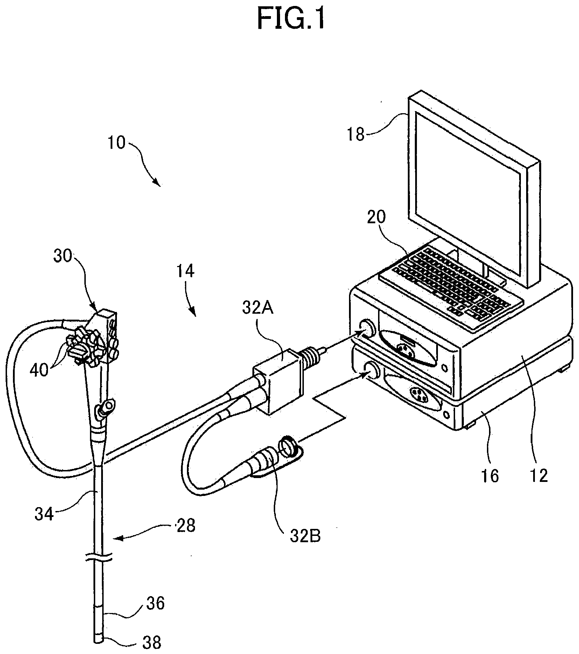

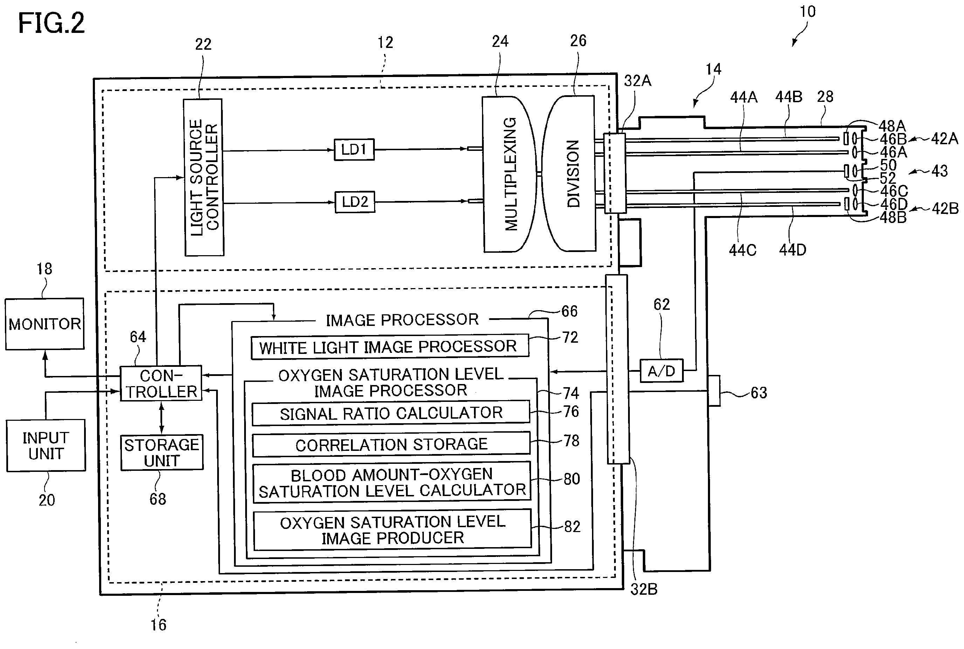

Fig. 1 is an external view of an embodiment illustrating a configuration of the endoscopic diagnosis system according to the invention; Fig. 2 is a block diagram of a first embodiment illustrating an internal configuration of that system shown in Fig. 1. As illustrated in these figures, the endoscopic diagnosis system 10 comprises a light source device 12 for emitting illumination light having a given range of wavelength; an endoscope device 14 for guiding light emitted from the light source device 12, illuminating a subject's region under observation with the illumination light, and imaging, for example, the reflected light; a processor 16 for image-processing the image signal acquired by the endoscope device 14; a monitor 18 for displaying, for example, the endoscopic image obtained through image processing by the processor 16; and an input unit 20 for receiving input operations.

The endoscopic diagnosis system 10 is capable of a white light observation mode in which a subject is illuminated by white light, and the reflected light is imaged to produce a white light image, which is displayed and observed; and an oxygen saturation level observation mode in which the blood hemoglobin oxygen saturation level calculated based on an image signal corresponding to three or more beams of reflected light having different wavelength ranges is displayed as pseudo color image for observation. The observation mode is switched as appropriate according to an instruction entered by a selector switch 63 of the endoscope device 14 or the input unit 20.

The light source device 12 comprises a light source controller 22, two kinds of laser light sources LD1, LD2 for emitting laser beams having different wavelength ranges, a combiner 24, and a coupler 26.

According to this embodiment, the laser light sources LD1, LD2 emit narrowband light beams having given wavelength ranges with central wavelengths of 473 nm and 445 nm (e.g., central wavelength +/- 10 nm), respectively. The laser light source LD1 is provided to acquire a narrowband light image for oxygen saturation level observation; the laser light source LD2 is provided for white light observation and emits excitation light to generate white light (pseudo white light) from a fluorescent body provided on the tip portion of the endoscope described later.

The on/off control and light amount control of the laser light sources LD1, LD2 are made independently between these light sources by the light source controller 22 controlled by a controller 64 of the processor 16.

The laser light sources LD1, LD2 may be constituted using, among others, broad area type InGaN-based laser diodes as well as InGaNas-based laser diodes and GaNas-based laser diodes.

The laser beams emitted from the laser light sources LD1, LD2 are passed through condenser lenses (not shown) to enter their respective optical fibers, combined by the combiner 24, a multiplexer, and divided into four channels of light beams by the coupler 26, a branching filter, before being transmitted to a connector unit 32A. The configuration is not limited this way; the laser beams from the laser light sources LD1, LD2 may be directly transmitted to the connector unit 32A in lieu of through the combiner 24 and the coupler 26.

The endoscope device 14 is an electronic endoscope instrument comprising an optical system for illumination for emitting four channels (beams) of illumination light from the tip of the endoscope inserted into the inside of the subject's body and one channel of optical imaging system for imaging the region under observation. The endoscope device 14 comprises an endoscope 28, an operating unit 30 for bending the tip portion thereof and performing an operation for observation among others, and connector units 32A, 32B for detachably connecting the endoscope device 14 to the light source device 12 and the processor 16.

The endoscope 28 comprises a flexible portion 34 having a flexibility, a bending portion 36, and a scope tip portion 38.

The bending portion 36 is provided between the flexible portion 34 and the scope tip portion 38 and is so configured as to be bendable by rotating an angle knob 40 provided on the operating unit 30. The bending portion 36 can be bent in any direction and to any angle according to, for example, the subject's site for which the endoscope device 14 is used, so that the scope tip portion 38 may be directed toward a desired site for observation.

At the scope tip portion 38 are provided two illumination windows 42A, 42B for emitting light to a region under observation and one observation window 43 for imaging, for example, the light reflected from the region under observation as illustrated in Fig. 2.

Behind and on the inside of the illumination window 42A are provided two channels of optical fibers 44A, 44B. The optical fibers 44A, 44B extend from the light source device 12 through the connector unit 32A to the scope tip portion 38. The optical fiber 44A has at its tip portion (its end closer to the illumination window 42A) an optical system including, for example, a lens 46A. The optical fiber 44B has at its tip portion a fluorescent body 48A, and beyond the fluorescent body 48A is provided an optical system including, for example, a lens 46B.

Behind and on the inside of the illumination window 42B are likewise provided two channels of optical fibers: an optical fiber 44C having at its tip portion an optical system including, for example, a lens 46C; and an optical fiber 44D having at its tip portion an optical system including, for example, a fluorescent body 48B and a lens 46D.

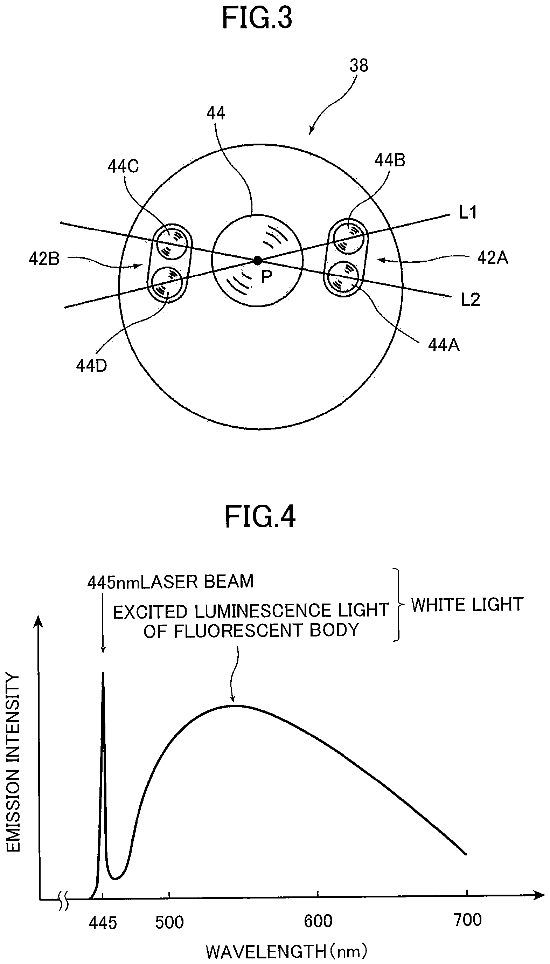

Fig. 3 is a front view of the tip portion of the endoscope. The illumination windows 42A, 42B are positioned on opposite side of the observation window 43. The four optical fibers 44A to 44D housed in the illumination windows 42A, 42B are provided in such alternate positions that a straight line L1 connecting the optical fibers 44B, 44D equipped with the fluorescent bodies 48A, 48B and a straight line L2 connecting the optical fibers 44A, 44C without fluorescent bodies intersect at a central point P of the observation window 43. Such arrangement of the optical fibers 44A to 44D prevents uneven illumination.

The fluorescent body 48A comprises a plurality of kinds of fluorescent substances that emit green to yellow light when excited upon absorbing part of the blue laser beam emitted from the laser light source LD2 (e.g., YAG-based fluorescent substance or BAM (BaMgAl₁₀O₁₇)-based fluorescent substance. When the excitation light for white light observation illuminates the fluorescent body 48A, the green to yellow light generated by excited luminescence light (fluorescence) emitted from the fluorescent body 48A blends with part of the blue laser beam that is transmitted without being absorbed by the fluorescent body 48A to generate white light (pseudo white light).

Fig. 4 shows a graph illustrating an emission spectrum of a blue laser beam emitted from the blue laser light source and of light obtained through wavelength conversion of blue laser beam by the fluorescent body. The blue laser beam emitted from the laser light source LD2 is represented by an emission line having a central wavelength of 445 nm; excited luminescence light excited by the blue laser beam and emitted from the fluorescent body 48A has a spectral intensity distribution where the light emission intensity increases in a wavelength range of about 450 nm to 700 nm. The excited luminescence light and the blue laser beam combines to produce pseudo white light as described above. The same applies to the fluorescent body 48B.

For the purpose of the invention, the white light is not limited to light containing strictly all the wavelength components of visible light but need only contain light having a specific wavelength range such as, for example, light having reference colors such as red, green, and blue, as well as the above pseudo white light. Thus, the white light herein broadly also includes, for example, light containing wavelength components corresponding to green to red light and light containing wavelength components corresponding to blue to green light

The optical system for illumination comprising the illumination window 42A and the optical system for illumination comprising the illumination window 42B have comparable configurations and effects, so that the illumination windows 42A, 42B basically emit equal illumination light simultaneously.

Behind the observation window 43 is installed an optical system including, for example, an object lens unit 50 for introducing image light from the subject's region under observation and, further behind the object lens unit 50 is installed an image sensor 52 such as a CCD (Charge Coupled Device) image sensor and a CMOS (Complementary Metal-Oxide Semiconductor) image sensor for acquiring image information on the subject's region under observation.

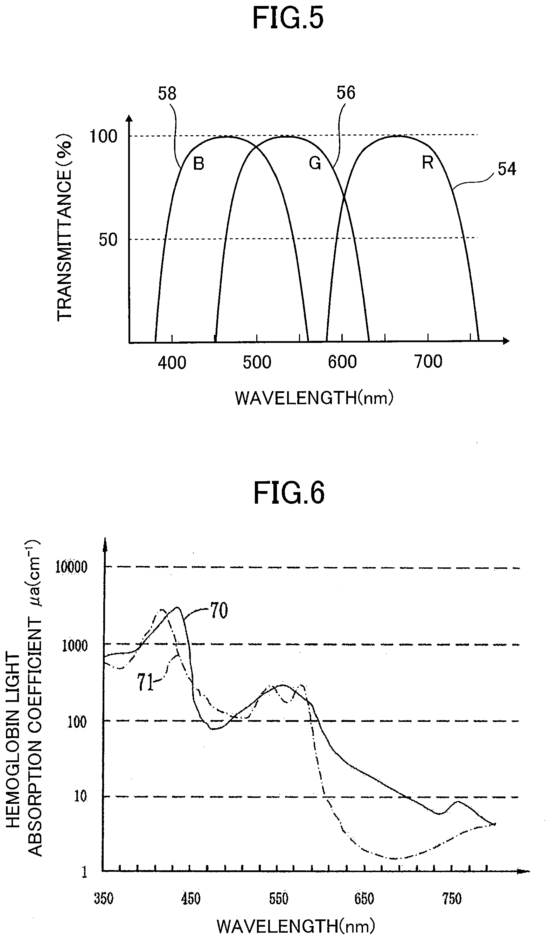

The image sensor 52 receives light from the object lens unit 50 with a light receiving surface (imaging surface), photoelectrically converts the received light into an imaging signal (analog signal), and outputs the imaging signal. The image sensor 52 in this embodiment is a color CCD image sensor whose light receiving surface comprises color filters, red filters 54, green filters 56, and blue filters 58, respectively having spectral transmittances as illustrated in Fig. 5; a red pixel, a green pixel, and a blue pixel form one set of pixels, and a plurality of sets of pixels are arranged in the form of matrix.

Since white light has a wavelength range of about 470 nm to 700 nm, the color filters composed of the red filters 54, the green filters 56, and the blue filters 58 transmit light corresponding to their respective spectral transmittances, and imaging signals of the red pixels, the green pixels, and the blue pixels are outputted. On the other hand, the laser beam emitted from the semiconductor laser LD1 has a central wavelength of 473 nm and, therefore, the reflected light thereof is transmitted only through the blue filters 58, so that an imaging signal of only the blue pixels is outputted.

The four channels of light transmitted from the light source device 12 are guided through the respective optical fibers 44A to 44D to the scope tip portion 38, and the guided light or the white light generated when the guided light illuminates the fluorescent bodies 48A, 48B and emitted from the fluorescent bodies 48A, 48B is emitted from the illumination windows 42A, 42B provided at the scope tip portion 38 toward the subject's region under observation. An image of the region under observation illuminated by the illumination light is formed on the light receiving surface of the image sensor 52 through, for example, the object lens unit 50, and imaged through photoelectric conversion by the image sensor 52.

The image sensor 52 outputs an imaging signal (analog signal) of the subject's imaged region under observation. An imaging signal of each image outputted from the image sensor 52 (analog signal) travels through a scope cable 60 to enter an A/D converter 62. The A/D converter 62 converts the analog imaging signal supplied from the image sensor 52 to a digital image signal corresponding in voltage level to the analog signal. The image signal obtained through the conversion passes through the connector unit 32B to enter an image processor 66 of the processor 16.

The operating unit 30 and the endoscope 28 contain therein a forceps channel for inserting, for example, a tissue collecting tool, air/water supply channels, and other channels, not shown.

The processor 16 comprises the controller 64, the image processor 66, and a storage unit 68. The controller 64 is connected to the monitor 18 and the input unit 20.

The controller 64 controls the operations of the image processor 66, the light source controller 22 of the light source device 12, and the monitor 18 according to instructions such as observation mode instruction entered by a selector switch 63 provided in the endoscope device 14 or the input unit 20.

The image processor 66 performs a given image processing on the image signal entered from the endoscope device 14 according to the observation mode under the control by the controller 64. The image processor 66 comprises a white light image processor 72 and an oxygen saturation level image processor 74.

In the white light observation mode, the white light image processor 72 performs a given image processing appropriate for the white light image on the image signal entered from the endoscope device 14 and outputs a white light image signal.

In the oxygen saturation level observation mode, the oxygen saturation level image processor 74 calculates information on the subject's blood amount and the blood hemoglobin oxygen saturation level based on the image signal entered from the endoscope device 14 and outputs an oxygen saturation level image signal for displaying the oxygen saturation level distribution in pseudo colors based on the calculated oxygen saturation level information. The oxygen saturation level image processor 74 comprises a signal ratio calculator 76, a correlation storage 78, a blood amount-oxygen saturation level calculator 80, and an oxygen saturation level image producer 82.

The signal ratio calculator 76 determines a blood vessel region based on the difference between the image signal for the blood vessel portion and the image signal for the other portions from the image signal entered from the endoscope device 14 The signal ratio calculator 76 obtains signal ratios S1/S3 and S2/S3 for pixels in the same position in the blood vessel region, where S1 and S2 respectively denote image signals corresponding to the reflected light of two narrowband light having a wavelength range where the order of magnitude of light absorption coefficients (absorbances) reverses between reduced hemoglobin and oxygenated hemoglobin according to the blood hemoglobin oxygen saturation level, and S3 denotes an image signal corresponding to the reflected light of one narrowband light having a wavelength range where the light absorption coefficients coincide.

The correlation storage 78 stores a correlation between the signal ratios S1/S3 and S2/S3 on the one hand and the blood amount and the oxygen saturation level on the other hand. That correlation is one where the blood vessel contains hemoglobin exhibiting light absorption coefficients as shown in Fig. 6 and is obtained by analyzing, for example, a number of image signals accumulated through diagnoses hitherto made.

As illustrated in Fig. 6, the blood hemoglobin has a light absorption characteristic having a light absorption coefficient µa changing according to the wavelength of light used for illumination. The light absorption coefficient µa denotes an absorbance representing the magnitude of light absorption by light. A reduced hemoglobin 70 and an oxygenated hemoglobin 71 have different light absorption characteristics such that they have different absorbances except for the isosbestic points at which both exhibit the same absorbance (intersections of light absorption characteristics curves of hemoglobin 70 and 71 in Fig. 6).

Generally, the distribution illustrated in Fig. 6 changes into a non-linear line depending on the site of the subject and, therefore, needs to be previously obtained by, for example, measuring an actual body tissue or conducting a simulation of light propagation.

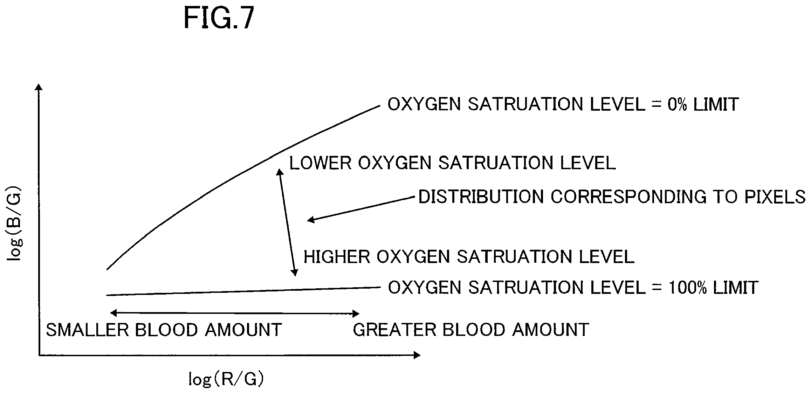

Fig. 7 is a graph illustrating a correlation between signal ratios B/G and R/G on the one hand and blood amount and oxygen saturation level on the other hand. In the graph, the horizontal axis shows log(R/G); the vertical axis shows log(B/G). The signal ratio R/G corresponds to the signal ratio S1/S3; the signal ratio B/G corresponds to the signal ratio S2/S3. As illustrated in the graph, the signal ratio R/G changes depending on the blood amount, increasing with the blood amount. The signal ratio B/G changes depending on both the blood amount and the oxygen saturation level. The signal ratio B/G increases with the blood amount and increases as the oxygen saturation level decreases.

The blood amount-oxygen saturation level calculator 80 calculates the blood amount and the oxygen saturation level corresponding to the signal ratios S1/S3 and S2/S3 calculated by the signal ratio calculator 76 based on the correlation stored in the correlation storage 78.

The oxygen saturation level image producer 82 has a color map where the magnitude of the oxygen saturation level is represented by color information. A color table is selectable and selected according to an instruction entered from the input unit 20 to suit the site under observation such as, for example, stomach, duodenum, or small intestine. Using the color map, the oxygen saturation level image producer 82 determines color information corresponding to the oxygen saturation level calculated by the blood amount-oxygen saturation level calculator 80. Upon determining the color information for all the pixels in the blood vessel region, the oxygen saturation level image producer 82 incorporates the color information in the image signal of the white light image in order to produce an oxygen saturation level image signal incorporating blood hemoglobin oxygen saturation level for display in pseudo colors.

The image signal processed by the image processor 66 is supplied to the controller 64, which produces an endoscopic observation image from this processed image and other information. The endoscopic observation image is displayed on the monitor 18 and, where necessary, stored in the storage unit 68 comprising a memory and a storage device.

Methods of calculating the blood amount and the oxygen saturation level will now be described.

When light enters a mucous tissue of a subject, part of the light is absorbed by the blood vessel, and part of the light that is not absorbed returns as reflected light. In the process, as the blood vessel depth increases, so does the scattering effects produced by the tissue lying above the blood vessel.

Light having a wavelength in a range of 470 nm to 700 nm has a small scattering coefficient and a small wavelength dependence. Accordingly, use of light having such range of wavelength for illumination enables information on the blood amount and the oxygen saturation level to be obtained while reducing the effects produced by the blood vessel depth. Thus, the endoscopic diagnosis system 10 calculates blood hemoglobin oxygen saturation level by using image signals corresponding to three or more reflected light having different wavelengths in a range of 460 nm to 700 nm including reflected light having two or more wavelength ranges where the light absorption coefficient changes according to the blood hemoglobin oxygen saturation level and reflected light having one or more wavelength ranges where light absorption coefficient does not changes.

From the wavelength dependence of the light absorption coefficient of the blood hemoglobin, the following holds:
- The light absorption coefficient changes greatly with the oxygen saturation level in a wavelength range close to 470 nm, say in a wavelength range of blue light of a central wavelength of 470 nm +/- 10 mm.
- The light absorption coefficient, when averaged in the wavelength range of green light of 540 nm to 580 nm, is not readily affected by the oxygen saturation level.
- In a wavelength range of red light of 590 nm to 700 nm, the light absorption coefficient appears to greatly change depending on the oxygen saturation level, but since the light absorption coefficient itself is extremely small, the ultimate effects produced by the oxygen saturation level are small.

From the reflectance spectrum from a mucous membrane, the following holds:
- It may be said that almost no effects are produced by hemoglobin in a wavelength of red light, but in a wavelength of red light, where absorption takes place, the difference between reflectance in a wavelength range of green light and reflectance in a wavelength range of red light increases with the blood amount, which corresponds to the blood vessel diameter or blood vessel density.
- The difference between reflectance in a wavelength close to 470 nm and reflectance in a wavelength of green light increases as the oxygen saturation level decreases and increases with the blood amount.

Accordingly, the signal ratio B/G of the image signal B of blue pixels to the image signal G of green pixels changes depending on both the oxygen saturation level and the blood amount; the signal ratio R/G of the image signal R of red pixels to the image signal G of green pixels changes depending mostly on the blood amount only. Using this nature, the oxygen saturation level and the blood amount can be separated from the spectral images ranging over three wavelength ranges containing a wavelength close to 470 nm and wavelength ranges of green and red light, and thereby accurately calculated. The graph produced based on the above is the graph of Fig. 7 referred to earlier illustrating a correlation between signal ratios B/G and R/G on the one hand and blood amount and oxygen saturation level on the other hand.

Next, the operation of the endoscopic diagnosis system 10 will be described.

The operation of the white light observation mode will be described first.

An instruction related to, for example, the observation mode is entered from the selector switch 63 of the endoscope device 14 or the input unit 20 to the controller 64 of the processor 16 to select the white light observation mode.

In the white light observation mode, the controller 64 of the processor 16 controls the operation of the light source controller 22 of the light source device 12 so as to turn off the laser light source LD1 and turn on the laser light source LD2, which emits two channels of excitation light for white light observation mode.

In the endoscope device 14, the two channels of excitation light for the white light observation mode emitted from the light source device 12 are guided through the optical fibers 44B, 44D to the fluorescent bodies 48A, 48B provided at the scope tip portion 38. Then, white light is emitted from the fluorescent bodies 48A, 48B and, passing through the lenses 46B, 46D, emitted from the illumination windows 42A, 42B to illuminate the subject's region under observation. The reflected light from the region under observation is condensed by the object lens unit 50, undergoes photoelectric conversion by the image sensor 52, and is outputted as imaging signal (analog signal) of a white light image.

The imaging signal (analog signal) of the white light image is converted into an image signal (digital signal) by the A/D converter 62 and undergoes a given image processing suited to the white light image by the white light image processor 72 of the image processor 66 according to the observation mode, whereupon a white light image signal is outputted. Then, the controller 64 generates a white light image from the white light image signal, and the white light image is displayed on the monitor 18.

The operation in the white light observation mode will now be described.

First, the observation mode is switched from the normal light image mode to the oxygen saturation level observation mode. In the oxygen saturation level observation mode, the white light image signal as of the time the observation mode is switched is stored in the storage unit 68 as reference image used to produce the oxygen saturation level image. The input unit 20 is operated to specify information on a site currently under observation such as stomach, duodenum, and small intestine. Then, the oxygen saturation level image producer 82 selects a color table according to the site under observation.

In the oxygen saturation level observation mode, illumination light having a different illumination pattern is emitted in each frame, two frames constituting one set. First, the LD1 is turned on and the LD2 is turned off in a first frame, so that two channels of laser beams for oxygen saturation level observation are emitted from the light source device 12.

In the endoscope device 14, the two channels of laser beams for oxygen saturation level observation emitted from the light source device 12 are guided through the optical fibers 44A, 44C to the scope tip portion 38 and emitted from the illumination windows 42A, 42B through the lenses 46A, 46C to illuminate the subject's region to be observed. The reflected light from the region under observation is condensed by the object lens unit 50, undergoes photoelectric conversion by the image sensor 52, and is outputted as imaging signal (analog signal) of a narrowband light image. The imaging signal (analog signal) of a narrowband light image is converted into an image signal (digital signal) by the A/D converter 62 and provisionally stored in the storage unit 68 by the control of the controller 64.

Then in a second frame, the LD1 is turned off and the LD2 is turned on, so that two channels of excitation light for white light observation are emitted from the light source device 12. In this operation, the endoscope device 14 operates in the same manner as in the white light observation mode, storing the white light image signal in the storage unit 68.

Let B1, G1, and R1 be the image signals obtained in the first frame, and let B2, G2, and R2 be the image signals obtained in the second frame. B1 is an image signal of an image of an image acquired by imaging using monochromatic illumination having a central wavelength of 473 nm; G2 is an image signal of an image acquired by imaging using spectral illumination by excitation light emitted from the fluorescent bodies 48A, 48B having a wavelength range of mainly 540 nm to 580 nm; and R2 is likewise an image signal of an image acquired by imaging using spectral illumination having a wavelength range of mainly 590 nm to 700 nm. B1 and R2 are image signals corresponding to reflected light having two wavelength ranges where the light absorption coefficient changes according to the blood hemoglobin oxygen saturation level; G2 is an image signal corresponding to reflected light having one wavelength range where the light absorption coefficient does not change.

Upon acquisition of the image signals in the first and the second frame, the signal ratio calculator 76 first determines a blood vessel region containing blood vessels from the narrowband light image in the first frame and the white light image signal in the second frame stored in the storage unit 68. Then, for the pixel in the same position in the blood vessel region, the signal ratio calculator 76 calculates the signal ratio B1/G2 between the image signal B1 of blue pixels in the first frame and the image signal G2 of green pixels in the second frame and the signal ratio R2/G2 between the image signal G2 of green pixels in the second frame and the image signal R2 of red pixels in the second frame.

As described above, the signal ratio B1/G2 changes depending on both the oxygen saturation level and the blood amount; the signal ratio R2/G2 changes depending mainly on the blood amount only. The blood amount-oxygen saturation level calculator 80 calculates information on the blood amount and the oxygen saturation level corresponding to the signal ratios B1/G2 and R2/G2 based on the correlation between the signal ratios B1/G2 and R2/G2 on the one hand and the blood amount and the oxygen saturation level on the other hand stored in the correlation storage 78 illustrated in Fig. 7.

Upon the blood amount and the oxygen saturation level being obtained, the oxygen saturation level image producer 82 determines color information corresponding to the oxygen saturation level based on a selected color table. The above procedure is repeated to obtain the blood amount and the oxygen saturation level for all the pixels in the blood vessel region and determine the color information corresponding to the oxygen saturation level. Then, when the oxygen saturation level and the corresponding color information have been obtained for all the pixels in the blood vessel region, the oxygen saturation level image producer 82 reads out the white light image signal, which is used as reference image, from the storage unit 68 and incorporates the color information in the white light image to produce the oxygen saturation level image signal. The oxygen saturation level image signal thus produced is stored in the storage unit 68.

The controller 64 reads out the oxygen saturation level image signal from the storage unit 68 and displays the oxygen saturation level image in pseudo colors on the monitor 18 based on the read oxygen saturation level image signal.

As described above, the endoscopic diagnosis system 10 is capable of accurately calculating oxygen saturation level information considering the blood amount while reducing the effects produced by the blood vessel depth and displaying the oxygen saturation level distribution as pseudo color image.

Next, a second embodiment will be described.

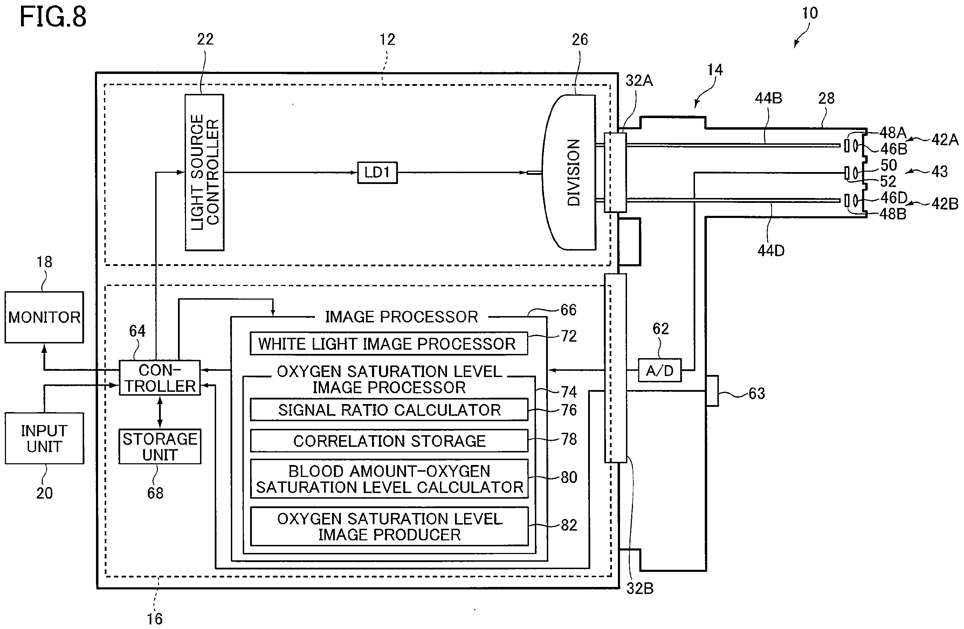

Fig. 8 is a block diagram of the second embodiment illustrating an internal configuration of the endoscopic diagnosis system shown in Fig. 1. In the endoscopic diagnosis system illustrated in that drawing, the light source device 12 comprises the light source controller 22, the laser light source LD1, and the coupler 26.

According to this embodiment, the laser light source LD1 emits a narrowband light beam having a given wavelength range with a central wavelength of 473 nm (e.g., central wavelength +/- 10 nm). The laser light source LD1 is provided to acquire a narrowband light image for oxygen saturation level observation and also emit excitation light in order to generate white light (pseudo white light) from fluorescent bodies 48A, 48B for white light observation.

Otherwise, the configuration of the light source device 12 is the same as in the endoscopic diagnosis system 10 according to the first embodiment illustrated in Fig. 2.

In the light source device 12, the light source controller 22 performs ON/OFF control and light amount control of the laser light source LD1, and the laser beam emitted from the laser light source LD1 is divided by the coupler 26 into two channels of light, which are transmitted to the connector unit 32A.

Subsequently, the endoscope device 14 comprises an optical system for illumination for emitting two channels (beams) of light from the tip portion of the endoscope.

Behind and on the inside of the illumination window 42A of the endoscope device 14 are provided one channel of optical fiber 44B having an optical system including, for example, the fluorescent body 48A and the lens 46B at the tip portion; behind and on the inside of the illumination window 42B is likewise provided one channel of optical fiber 44D having an optical system including, for example, the fluorescent body 48B and the lens 46D at the tip portion. Both have equivalent configurations and effects, so that the illumination windows 42A, 42B basically emit equivalent illumination light simultaneously.

The two channels of light emitted from the light source device 12 are guided through the respective optical fibers 44B, 44D to the scope tip portion 38. When the laser beam leaving the laser light source LD1 illuminates the fluorescent body 48A, the green to yellow excited luminescence light emitted from the fluorescent body 48A blends with the part of the laser beam that is transmitted through the fluorescent body 48A without being thereby absorbed to generate white light (pseudo white light). The same applies to the fluorescent body 48B.

Otherwise, the configurations of the endoscope device 14 and the processor 16 are the same as in the endoscopic diagnosis system 10 according to the first embodiment illustrated in Fig. 2.

Next, the operation of the second embodiment of the endoscopic diagnosis system will be described.

This embodiment shares the same operation in the white light observation mode with the first embodiment.

In the oxygen saturation level observation mode, the LD1 is turned on to emit two channels of laser beams from the light source device 12. While the first embodiment requires switching between the light sources every two frames to calculate the oxygen saturation level, the second embodiment may calculate the oxygen saturation level without switching between the light sources every frame. Capability of acquiring spectral information in a single frame makes the system less liable to be affected by the subject's movements.

Now, let B, G, and R be image signals obtained in one frame, then B contains an image signal of the excitation light having a central wavelength of 473 nm and an image signal of a small amount of excited luminescence light emitted from the fluorescent bodies 48A, 48B. G contains an image signal of an image acquired by imaging using spectral illumination by excited luminescence light emitted from the fluorescent bodies 48A, 48B having a wavelength range of mainly 540 nm to 580 nm and an image signal of an image acquired by imaging using a small amount of excitation light; R is an image signal of an image acquired by imaging using spectral illumination having a wavelength range of 590 nm to 700 nm.

Preferably, the color CCD image sensor used has color filters that minimize the components of the image signal of the excited luminescence light mixed into the image signal of B and the components of the excitation light mixed into the image signal of G.

Accordingly, the information on the blood amount and the oxygen saturation level corresponding to the signal ratios B/G and R/G based on the correlation between the signal ratios B/G and R/G on the one hand and the blood amount and the oxygen saturation level on the other hand in the same manner as in the first embodiment.

Next, a third embodiment will be described.

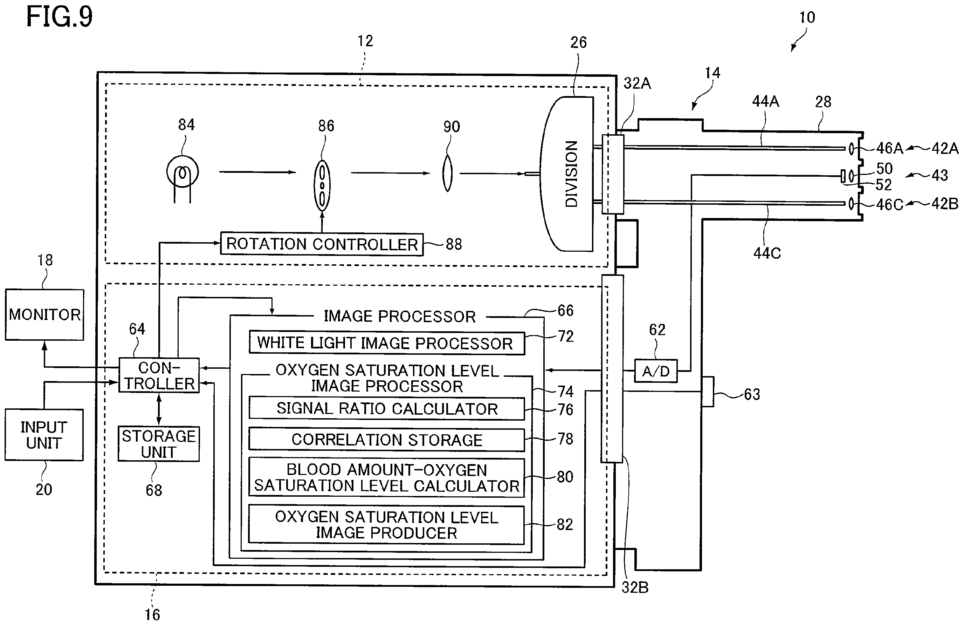

Fig. 9 is a block diagram of the third embodiment illustrating an internal configuration of the endoscopic diagnosis system shown in Fig. 1. The light source device 12 of the endoscopic diagnosis system illustrated in that drawing comprises a white light source 84, a narrowband filter 86, a rotation controller 88, a lens 90, and the coupler 26.

The white light source 84 may, for example, be on and emits white light whenever the light source device 12 is on. Examples of the white light source 84 include white light emitting lamps such as a xenon lamp, a fluorescent lamp, and a mercury lamp and any other light source that emits white light.

The narrowband filter 86 is a band pass filter that filters the white light emitted from the white light source 84, transmitting light having a given wavelength range. The narrowband filter 86 has the shape of a disk and comprises a light transmitting portion that allows white light to pass as it is and a first to a third light filtering portion that transmit first narrowband light having a wavelength of 460 nm to 480 nm, second narrowband light having a wavelength of 540 nm to 580 nm, and third narrowband light having a wavelength of 590 nm to 700 nm. The narrowband filter 86 is provided on the optical path between the white light source 84 and the lens 90 in a perpendicular position with respect to the optical path and rotated as necessary by a motor, not shown, under the control by the rotation controller 88.

The rotation controller 88 controls the rotation of the narrowband filter 86 under the control by the controller 64 of the processor 16. In the white light observation mode, the rotation of the narrowband filter 86 is so controlled that the light transmitting portion is aligned with the optical path to allow the white light to pass as it is. In the oxygen saturation level observation mode, the rotation of the narrowband filter 86 is so controlled that the first to the third light filtering portion are aligned sequentially with the optical path in each frame of the first to the third frame to allow the first to the third narrowband light to pass sequentially in each frame.

In the light source device 12, the white light emitted from the white light source 84 undergoes filtering through the narrowband filter 86 under the control by the rotation controller 88, is condensed by the lens 90, and divided by the coupler 26 into two channels of light before being transmitted to the connector unit 32A.

The endoscope device 14 comprises an optical system for illumination for emitting two channels (beams) of light from the tip portion of the endoscope.

Behind and on the inside of the illumination window 42A of the endoscope device 14 are provided one channel of optical fiber 44A having an optical system including, for example, the lens 46A at the tip portion; behind and on the inside of the illumination window 42B is likewise provided one channel of optical fiber 44C having an optical system including, for example, the lens 46C at the tip portion. Both have equivalent configurations and effects, so that the illumination windows 42A, 42B basically emit equivalent illumination light simultaneously.

The two channels of light emitted from the light source device 12 are guided through the respective optical fibers 44A, 44C to the scope tip portion 38 and emitted therefrom to illuminate the subject's region under observation. The image sensor 52 according to this embodiment is a monochromatic CCD image sensor and outputs an imaging signal having a luminance corresponding to the reflected light of the illumination light illuminating the subject.

The processor 16 has the same configuration as in the first embodiment.

Next, the operation of the third embodiment of the endoscopic diagnosis system will be described.

In the white light observation mode, the rotation controller 88 controls the rotation of the narrowband filter 86 so as to align the light transmitting portion with the optical path so that the light source device 12 emits two channels of white light.

In the endoscope device 14, the two channels of white light emitted from the light source device 12 are guided through the optical fibers 44A, 44C to the scope tip portion 38 and emitted respectively from the illumination windows 42A, 42B through the lenses 46A, 46C to illuminate the subject's region to be observed. The operation to follow is the same as in the first embodiment except that a monochromatic white light image is imaged in lieu of a color white light image.

In the oxygen saturation level observation mode, the wavelength ranges of the illumination light are switched every frame using the narrowband filter 86, three frames constituting one set. More specifically, the rotation controller 88 controls the rotation of the narrowband filter 86 to sequentially align the first to the third filtering portion with the optical path, so that the first to the third narrowband light are sequentially emitted from the light source device 12 in each frame and guided to illuminate the subject sequentially.

Now, let B, G, and R be image signals obtained in the first to the third frame. Then, B is an image signal having a wavelength range of 460 nm to 480 nm; G is an image signal having a wavelength range of 540 nm to 580 nm; and R is an image signal having a wavelength range of 590 nm to 700 nm.

Accordingly, the information on the blood amount and the oxygen saturation level corresponding to the signal ratios B/G and R/G can be calculated based on the correlation between the signal ratios B/G and R/G on the one hand and the blood amount and the oxygen saturation level on the other hand in the same manner as in the first embodiment.

Next, a fourth embodiment will be described.

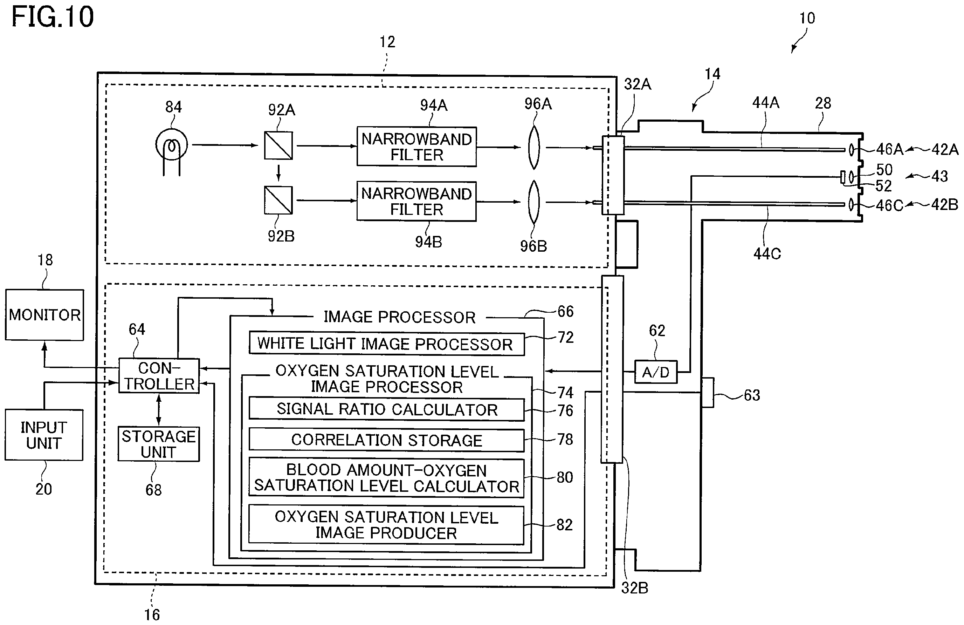

Fig. 10 is a block diagram of the fourth embodiment illustrating an internal configuration of the endoscopic diagnosis system shown in Fig. 1. The light source device 12 of the endoscopic diagnosis system illustrated in that drawing comprises a white light source 84, a half mirror 92A, a reflection mirror 92B, narrowband filters 94A, 94B, and lenses 96A, 96B. In that drawing, the portion related to acquisition of the white light image is not included for simplicity.

The white light source 84 is the same as in the third embodiment illustrated in Fig. 9. The white light emitted from the white light source 84 is divided by the half mirror 92A into two channels of equivalent white light. The white light passing through the half mirror 92A is allowed to enter the narrowband filter 94A. On the other hand, the white light reflected and bent by 90° by the half mirror 92A in a downward direction as seen in the drawing is further reflected and bent by 90° by the reflection mirror 92B in a rightward direction as seen in the drawing to enter the narrowband filter 94B.

The narrowband filters 94A, 94B are band pass filters for filtering the entered white light to transmit narrowband light having a wavelength range of 460 nm to 480 nm and a wavelength range of 540 nm to 700 nm, respectively.

In the light source device 12, the white light emitted from the white light source 84 is divided by the half mirror 92A and the reflection mirror 92B into two channels of equivalent white light, which undergo filtering through the narrowband filters 94A, 94B and are condensed by the lenses 96A, 96B before being transmitted to the connector unit 32A.

The endoscope device 14 is the same as in the third embodiment illustrated in Fig. 9 except that the image sensor 52 is a color CCD image sensor.

The two channels of light emitted from the light source device 12 are guided through the respective optical fibers 44A, 44C to the scope tip portion 38 and emitted therefrom to illuminate the subject's region under observation.

The processor 16 has the same configuration as in the first embodiment.

Next, the operation of the fourth embodiment of the endoscopic diagnosis system will be described.

In the oxygen saturation level observation mode, two kinds of narrowband light, narrowband light having a wavelength range of 460 nm to 480 nm and narrowband light having a wavelength range of 540 nm to 580 nm, are emitted simultaneously from the light source device 12 and guided to illuminate the subject simultaneously. Then, the reflected light returning from the subject is converted into an imaging signal by the color CCD image sensor, the image sensor 52, and converted into an image signal by the A/D converter 62.

Now, let B, G, and R be A/D-converted image signals. Then, B is an image signal having a wavelength range of 460 nm to 480 nm; G is an image signal having a wavelength range of mainly 540 nm to 580 nm; and R is an image signal having a wavelength range of 590 nm to 700 nm.

Accordingly, the information on the blood amount and the oxygen saturation level corresponding to the signal ratios B/G and R/G can be calculated based on the correlation between the signal ratios B/G and R/G on the one hand and the blood amount and the oxygen saturation level on the other hand in the same manner as in the first embodiment.

Next, a fifth embodiment will be described.

The fifth embodiment has the same configuration as the third embodiment illustrated in Fig. 9 except for the characteristics of the narrowband filter 86. In the light source device 14 of the endoscopic diagnosis system according to the fifth embodiment, the first to the third light filtering portion of the narrowband filter 86 transmit the first narrowband light having a wavelength of 530 nm to 550 nm, the second narrowband light having a wavelength of 555 nm to 565 nm, and the third narrowband light having a wavelength of 590 nm to 700 nm of the white light emitted from the white light source 84.

In the oxygen saturation level observation mode, the rotation controller 88 controls the rotation of the narrowband filter 86 so that the first to the third light filtering portion are sequentially aligned with the optical path in each of the first to the third frame. Accordingly, the first to the third narrowband light are sequentially transmitted in each frame.

Next, the operation of the fifth embodiment of the endoscopic diagnosis system will be described.

This embodiment shares the same operation in the oxygen saturation level observation mode with the third embodiment. Now, let G1, G2, and R be image signals obtained in the first to the third frame. Then, G1 is an image signal having a wavelength range of 530 nm to 550 nm; G2 is an image signal having a wavelength range of 555 nm to 565 nm; and R is an image signal having a wavelength range of 590 nm to 700 nm. G1 and R2 are image signals corresponding to reflected light having two wavelength ranges where the light absorption coefficient changes according to the blood hemoglobin oxygen saturation level; G2 is an image signal corresponding to reflected light having one wavelength range where the light absorption coefficient does not change. Accordingly, the signal ratio G1/G2 changes depending on the oxygen saturation level and the blood amount; the signal ratio R/G2 changes depending mainly on the blood amount.

Thus, the information on the blood amount and the oxygen saturation level corresponding to the signal ratios G1/G2 and R/G2 may be calculated as in the first embodiment based on the correlation between the signal ratios G1/G2 and R/G2 on the one hand and the blood amount and the oxygen saturation level on the other hand by replacing the two signal ratios B1/G2 and R/G2 in the first embodiment with the signal ratios G1/G2 and R/G2.

The combination of the kind of the light source (e.g., laser light source, white light source, a combination of a laser light source and a white light source) and the wavelength, the type of the image sensor (color or monochromatic), the illumination pattern of the illumination light (e.g., each frame, a set of frames), and the type of the optical systems for illumination (one beam, two beams, four beams, and so forth), among others, of the endoscope device may be changed as necessary, provided that the endoscope device is capable of acquiring image signals corresponding to three or more different reflected light having wavelengths in a range of 460 nm to 700 nm, including reflected light having two wavelength ranges where the light absorption coefficient changes according to the blood hemoglobin oxygen saturation level and reflected light having one wavelength range where the light absorption coefficient does not change.

The present invention is basically as described above.

While the invention has been described above in detail, the invention is by no means limited to the above embodiments, and various improvements and modifications may of course be made without departing from the claimed subject-matter.

## Claims

1. An endoscopic diagnosis system (10) comprising:
a light source device (12) for emitting light having a given wavelength range;
an endoscope device (14) having an image sensor (52), and for guiding the light emitted from the light source device (12), illuminating a subject with illumination light, and imaging reflected light of the illumination light with the image sensor (52) to acquire image signals;
a blood amount-oxygen saturation level calculating section (80) for calculating a blood amount and a blood hemoglobin oxygen saturation level of the subject based on the acquired image signals; and
an image display device (18) for displaying an oxygen saturation level distribution as pseudo color image based on information on the oxygen saturation level,
**characterized in that** the endoscope device (14) is adapted to acquire three or more image signals including a first image signal corresponding to reflected light having a wavelength range of 460 nm to 480 nm, a second image signal corresponding to reflected light having a wavelength range of 590 nm to 700 nm, and a third image signal corresponding to reflected light having a wavelength range including 540 nm to 580 nm, and
the blood amount-oxygen saturation level calculating section (80) comprises a correlation storage (78) having stored therein a correlation between a first signal ratio between the first image signal and the third image signal and a second signal ratio between the second image signal and the third image signal on the one hand and the blood amount and the oxygen saturation level on the other hand, the blood amount-oxygen saturation level calculating section (80) being adapted for calculating the first and the second signal ratio from the first to the third image signal and for calculating information on the blood amount and the oxygen saturation level corresponding to the calculated first and second signal ratios using the correlation stored in the correlation storage (78).

2. The endoscopic diagnosis system (10) according to claim 1, wherein,
the endoscope device (14) is adapted to illuminate in a first frame the subject with light having a central wavelength of 473 nm, and to image reflected light of the light with the image sensor (52) being a color image sensor to acquire the first image signal; and,
is adapted to guide, in a second frame, excitation light having a central wavelength of 445 nm and to illuminate a fluorescent body (48A, 48B) provided at a tip portion of an endoscope (28) to illuminate the subject with pseudo white light containing excitation light transmitted through the fluorescent body (48A, 48B) and excited luminescence light emitted from the fluorescent body (48A, 48B), thereby to image reflected light of the pseudo white light with the image sensor (52) being the color image sensor and acquire the second and the third image signal.

3. The endoscopic diagnosis system (10) according to claim 1, wherein the endoscope device (14) is adapted to guide excitation light having a central wavelength of 473 nm, to illuminate a fluorescent body (48A, 48B) provided at a tip portion of an endoscope (28) to illuminate the subject with pseudo white light containing excitation light transmitted through the fluorescent body (48A, 48B) and excited luminescence light emitted from the fluorescent body (48A, 48B), and to image reflected light of the pseudo white light with the image sensor (52) being a color image sensor thereby to acquire the first to the third image signal.

4. The endoscopic diagnosis system (10) according to claim 1, wherein the endoscope device (14) is adapted to filter white light emitted from a white light source (84) through a narrowband filter (86); and,
to illuminate in a first frame the subject with light having a wavelength range of 460 nm to 480 nm and to image reflected light of the light with the image sensor (52) being a monochromatic image sensor to acquire the first image signal;
to illuminate in a second frame the subject with light having a wavelength range of 540 nm to 580 nm and to image reflected light of the light with the image sensor (52) being the monochromatic image sensor to acquire the third image signal; and
to illuminate in a third frame the subject with light having a wavelength range of 590 nm to 700 nm and to image reflected light of the light with the image sensor (52) being the monochromatic image sensor to acquire the second image signal.

5. The endoscopic diagnosis system (10) according to claim 1, wherein the endoscope device (14) is adapted to filter white light emitted from a white light source (84) of the light source device (12) through a first and a second narrowband filter (94A, 94B), to illuminate the subject with light having a wavelength range of 460 nm to 480 nm and light having a wavelength range of 540 nm to 700 nm simultaneously, and to image reflected light of these lights with the image sensor (52) being a color image sensor to acquire the first to the third image signal.

6. An endoscopic diagnosis system (10) comprising:
a light source device (12) for emitting light having a given wavelength range;
an endoscope device (14) having an image sensor (52), and for guiding the light emitted from the light source device (12), illuminating a subject with illumination light, and imaging reflected light of the illumination light with the image sensor (52) to acquire image signals;
a blood amount-oxygen saturation level calculating section (80) for calculating a blood amount and a blood hemoglobin oxygen saturation level of the subject based on the acquired image signals; and
an image display device (18) for displaying an oxygen saturation level distribution as pseudo color image based on information on the oxygen saturation level,
**characterized in that** the endoscope device (14) is adapted to filter white light emitted from a white light source (84) of the light source device (12) through a narrowband filter (86); and in a first frame, to illuminate the subject with light having a wavelength range of 530 nm to 550 nm and to image reflected light of the light with the image sensor (52) being a monochromatic image sensor to acquire a first image signal, in a second frame, to illuminate the subject with light having a wavelength range including 555 nm to 565 nm and to image reflected light of the light with the image sensor (52) being the monochromatic image sensor to acquire a third image signal, and in a third frame, to illuminate the subject with light having a wavelength range of 590 nm to 700 nm and to image reflected light of the light with the image sensor (52) being the monochromatic image sensor to acquire a second image signal, and
the blood amount-oxygen saturation level calculating section (80) comprises a correlation storage (78) having stored therein a correlation between a first signal ratio between the first image signal and the third image signal and a second signal ratio between the second image signal and the third image signal on the one hand and the blood amount and the oxygen saturation level on the other hand, the blood amount-oxygen saturation level calculating section (80) being adapted for calculating the first and the second signal ratio from the first to the third image signal and for calculating information on the blood amount and the oxygen saturation level corresponding to the calculated first and second signal ratios using the correlation stored in the correlation storage (78).

## Patentansprüche

1. Endoskop-Diagnose-System (10) umfassend:
eine Lichtquelleneinrichtung (12) zum Emittieren von Licht eines gegebenen Wellenlängenbereichs;
Endoskop-Einrichtung (14) mit einem Bildsensor (52) und zum Führen des von der Lichtquelleneinrichtung (12) emittierten Lichts, zum Beleuchten eines Subjekts mit Beleuchtungslicht und zum Abbilden von reflektiertem Licht des Beleuchtungslichts mit einem Bildsensor (52) zum Erfassen von Bildsignalen;
einen Blutmenge-Sauerstoffsättigungslevel-Berechnungsabschnitt (80) zum Berechnen einer Blutmenge und eines Blut-Hämoglobin-Sauerstoffsättigungslevels des Subjekts, basierend auf den erfassten Bildsignalen; und
eine Bild-Anzeigeeinrichtung (18) zum Anzeigen einer Sauerstoffsättigungslevel-Verteilung als Pseudofarbbild, basierend auf Informationen über den Sauerstoffsättigungslevel,
**dadurch gekennzeichnet, dass** die Endoskop-Einrichtung (14) eingerichtet ist, drei oder mehr Bildsignale zu erfassen, umfassend ein erstes Bildsignal, das reflektiertem Licht mit einem Wellenlängenbereich von 460 nm bis 480 nm entspricht, ein zweites Bildsignal, das reflektiertem Licht mit einem Wellenlängenbereich von 590 nm bis 700 nm entspricht, und ein drittes Bildsignal, das reflektiertem Licht mit einem Wellenlängenbereich von 540 nm bis 580 nm entspricht, und
der Blutmenge-Sauerstoffsättigungslevel-Berechnungsabschnitt (80) einen Korrelationsspeicher (78) umfasst, mit einer darin gespeicherten Korrelation zwischen einem ersten Signalverhältnis zwischen dem ersten Bildsignal und dem dritten Bildsignal und einem zweiten Signalverhältnis zwischen dem zweiten Bildsignal und dem dritten Bildsignal einerseits und der Blutmenge und dem Sauerstoffsättigungslevel andererseits, wobei der Blutmenge-Sauerstoffsättigungslevel-Berechnungsabschnitt (80) eingerichtet ist zum Berechnen des ersten und des zweiten Signalverhältnisses aus dem ersten bis dritten Bildsignal und zum Berechnen von Information über die Blutmenge und den Sauerstoffsättigungslevel entsprechend den berechneten ersten und zweiten Signalverhältnissen unter Verwendung der in dem Korrelationsspeicher (78) gespeicherten Korrelation.

2. Endoskop-Diagnose-System (10) nach Anspruch 1, wobei
die Endoskop-Einrichtung (14) eingerichtet ist, in einem ersten Frame das Subjekt mit Licht mit einer Zentralwellenlänge von 473 nm zu beleuchten und das von dem Licht reflektierte Licht mit dem als Farbbildsensor ausgestalteten Bildsensor (52) abzubilden, um das erste Bildsignal zu erfassen; und
eingerichtet ist, in einem zweiten Frame Anregungslicht mit einer Zentralwellenlänge von 445 nm zu führen und einen Fluoreszenzkörper (48A, 48B) zu beleuchten, der in einem Spitze-Abschnitt eines Endoskops (28) vorgesehen ist, um das Subjekt mit Pseudoweißlicht zu beleuchten, das durch den Fluoreszenzkörper (48A, 48B) transmittiertes Anregungslicht und von dem Fluoreszenzkörper (48A, 48B) emittiertes angeregtes Lumineszenzlicht enthält, um dadurch von dem Pseudoweißlicht reflektiertes Licht mit dem als Farbbildsensor ausgestalteten Farbsensor (52) abzubilden und das zweite und das dritte Bildsignal zu erfassen.

3. Endoskop-Diagnose-System (10) nach Anspruch 1, wobei die Endoskop-Einrichtung (14) eingerichtet ist, Anregungslicht mit einer Zentralwellenlänge von 473 nm zu führen, um einen Fluoreszenzkörper (48A, 48B) zu beleuchten, der in einem Spitze-Abschnitt eines Endoskops (28) vorgesehen ist, um das Subjekt mit Pseudoweißlicht zu beleuchten, das durch den Fluoreszenzkörper (48A, 48B) transmittiertes Anregungslicht und von dem Fluoreszenzkörper (48A, 48B) emittiertes angeregtes Lumineszenzlicht enthält, um von dem Pseudoweißlicht reflektiertes Licht dadurch mit dem als Farbbildsensor ausgestalteten Farbsensor (52) abzubilden um das erste bis dritte Bildsignal zu erfassen.

4. Endoskop-Diagnose-System (10) nach Anspruch 1, wobei die Endoskop-Einrichtung (14) eingerichtet ist, von einer Weißlichtquelle (84) emittiertes Weißlicht durch ein schmalbandiges Filter (86) zu filtern; und
in einem ersten Frame das Subjekt mit Licht mit einem Wellenlängenbereich von 460 nm bis 480 nm zu beleuchten und das von dem Licht reflektierte Licht mit dem als monochromatischer Bildsensor ausgebildeten Bildsensor (52) abzubilden, um das erste Bildsignal zu erfassen; und
in einem zweiten Frame das Subjekt mit Licht mit einem Wellenlängenbereich von 540 nm bis 580 nm zu beleuchten und das von dem Licht reflektierte Licht mit dem als monochromatischer Bildsensor ausgebildeten Bildsensor (52) abzubilden, um das dritte Bildsignal zu erfassen; und
in einem dritten Frame das Subjekt mit Licht mit einem Wellenlängenbereich von 590 nm bis 700 nm zu beleuchten und das von dem Licht reflektierte Licht mit dem als monochromatischer Bildsensor ausgebildeten Bildsensor (52) abzubilden, um das zweite Bildsignal zu erfassen.

5. Endoskop-Diagnose-System (10) nach Anspruch 1, wobei die Endoskop-Einrichtung (14) eingerichtet ist, von einer Weißlichtquelle (84) der Lichtquelleneinrichtung (12) emittiertes Weißlicht durch ein erstes und ein zweites schmalbandiges Filter (94A, 94B) zu filtern, um das Subjekt mit Licht mit einem Wellenlängenbereich von 460 nm bis 480 nm und Licht mit einem Wellenlängenbereich von 540 nm bis 700 nm gleichzeitig zu beleuchten und um von diesen Lichtern reflektiertes Licht mit dem als Farbbildsensor ausgestalteten Bildsensor (52) abzubilden, um das erste bis dritte Bildsignal zu erfassen.

6. Endoskop-Diagnose-System (10) umfassend:
eine Lichtquelleneinrichtung (12) zum Emittieren von Licht eines gegebenen Wellenlängenbereichs;
Endoskop-Einrichtung (14) mit einem Bildsensor (52) und zum Führen des von der Lichtquelleneinrichtung (12) emittierten Lichts, zum Beleuchten eines Subjekts mit Beleuchtungslicht und zum Abbilden von reflektiertem Licht des Beleuchtungslichts mit einem Bildsensor (52) zum Erfassen von Bildsignalen;
einen Blutmenge-Sauerstoffsättigungslevel-Berechnungsabschnitt (80) zum Berechnen einer Blutmenge und eines Blut-Hämoglobin-Sauerstoffsättigungslevels des Subjekts, basierend auf den erfassten Bildsignalen; und
eine Bild-Anzeigeeinrichtung (18) zum Anzeigen einer Sauerstoffsättigungslevel-Verteilung als Pseudofarbbild, basierend auf Informationen über den Sauerstoffsättigungslevel,
**dadurch gekennzeichnet, dass** die Endoskop-Einrichtung (14) eingerichtet ist; von einer Weißlichtquelle (84) der Lichtquelleneinrichtung (12) emittiertes Weißlicht durch ein schmalbandiges Filter (86) zu filtern, und in einem ersten Frame das Subjekt mit Licht mit einem Wellenlängenbereich von 530 nm bis 550 nm zu beleuchten und das von dem Licht reflektierte Licht mit dem als monochromatischer Bildsensor ausgebildeten Bildsensor (52) abzubilden, um das erste Bildsignal zu erfassen; in einem zweiten Frame das Subjekt mit Licht mit einem Wellenlängenbereich von 555 nm bis 565 nm zu beleuchten und das von dem Licht reflektierte Licht mit dem als monochromatischer Bildsensor ausgebildeten Bildsensor (52) abzubilden, um das dritte Bildsignal zu erfassen; und in einem dritten Frame das Subjekt mit Licht mit einem Wellenlängenbereich von 590 nm bis 700 nm zu beleuchten und das von dem Licht reflektierte Licht mit dem als monochromatischer Bildsensor ausgebildeten Bildsensor (52) abzubilden, um das zweite Bildsignal zu erfassen; und
der Blutmenge-Sauerstoffsättigungslevel-Berechnungsabschnitt (80) einen Korrelationsspeicher (78) umfasst, mit einer darin gespeicherten Korrelation zwischen einem ersten Signalverhältnis zwischen dem ersten Bildsignal und dem dritten Bildsignal und einem zweiten Signalverhältnis zwischen dem zweiten Bildsignal und dem dritten Bildsignal einerseits und der Blutmenge und dem Sauerstoffsättigungslevel andererseits, wobei der Blutmenge-Sauerstoffsättigungslevel-Berechnungsabschnitt (80) eingerichtet ist zum Berechnen des ersten und des zweiten Signalverhältnisses aus dem ersten bis dritten Bildsignal und zum Berechnen von Information über die Blutmenge und den Sauerstoffsättigungslevel entsprechend den berechneten ersten und zweiten Signalverhältnissen unter Verwendung der in dem Korrelationsspeicher (78) gespeicherten Korrelation.

## Revendications

1. Système de diagnostic endoscopique (10), comprenant :
un dispositif de source lumineuse (12) pour émettre une lumière présentant une plage de longueurs d'onde donnée ;
un dispositif d'endoscope (14) présentant un capteur d'image (52), et pour guider la lumière émise à partir du dispositif de source lumineuse (12), éclairer un sujet avec une lumière d'éclairage, et imager une lumière réfléchie de la lumière d'éclairage avec le capteur d'image (52) afin d'acquérir des signaux d'image ;
une section de calcul de niveau de saturation en oxygène d'une quantité de sang (80) pour calculer une quantité de sang et un niveau de saturation en oxygène de l'hémoglobine du sang du sujet sur la base des signaux d'image acquis, et
un dispositif d'affichage d'image (18) pour afficher une distribution de niveau de saturation en oxygène comme image de pseudo-couleur sur la base d'informations sur le niveau de saturation en oxygène,
**caractérisé en ce que** le dispositif d'endoscope (14) est apte à acquérir trois signaux d'image, ou plus, incluant un premier signal d'image correspondant à une lumière réfléchie présentant une plage de longueurs d'onde allant de 460 nm à 480 nm, un deuxième signal d'image correspondant à une lumière réfléchie présentant une plage de longueurs d'onde allant de 590 nm à 700 nm, et un troisième signal d'image correspondant à une lumière réfléchie présentant une plage de longueurs d'onde allant de 540 nm à 580 nm, et
la section de calcul de niveau de saturation en oxygène d'une quantité de sang (80) comprend un stockage de corrélation (78) à l'intérieur duquel est stockée une corrélation, d'une part, entre un premier rapport de signaux entre le premier signal d'image et le troisième signal d'image et un second rapport de signaux entre le deuxième signal d'image et le troisième signal d'image, et d'autre part, entre la quantité de sang et le niveau de saturation en oxygène, la section de calcul de niveau de saturation en oxygène d'une quantité de sang (80) étant apte à calculer le premier et le second rapports de signaux à partir du premier au troisième signaux d'image, et à calculer les informations sur la quantité de sang et le niveau de saturation en oxygène correspondant aux premier et second rapports de signaux calculés à l'aide de la corrélation stockée dans le stockage de corrélation (78).

2. Système de diagnostic endoscopique (10) selon la revendication 1, dans lequel
le dispositif d'endoscope (14) est apte à éclairer, dans une première trame, le sujet avec une lumière présentant une longueur d'onde centrale de 473 nm, et à imager une lumière réfléchie de la lumière avec le capteur d'image (52) étant un capteur d'image couleur pour acquérir le premier signal d'image, et
est apte à guider, dans une deuxième trame, une lumière d'excitation présentant une longueur d'onde centrale de 445 nm et à éclairer un corps fluorescent (48A, 48B) prévu sur une portion de pointe d'un endoscope (28) afin d'éclairer le sujet avec une pseudo-lumière blanche contenant une lumière d'excitation transmise à travers le corps fluorescent (48A, 48B), et une lumière de luminescence excitée émise à partir du corps fluorescent (48A, 48B), afin ainsi d'imager la lumière réfléchie de la pseudo-lumière blanche avec le capteur d'image (52) étant le capteur d'image couleur et d'acquérir les deuxième et troisième signaux d'image.

3. Système de diagnostic endoscopique (10) selon la revendication 1, dans lequel le dispositif d'endoscope (14) est apte à guider une lumière d'excitation présentant une longueur d'onde centrale de 473 nm, afin d'éclairer un corps fluorescent (48A, 48B) prévu sur une portion de pointe d'un endoscope (28) afin d'éclairer le sujet avec une pseudo-lumière blanche contenant une lumière d'excitation transmise à travers le corps fluorescent (48A, 48B) et une lumière de luminescence excitée émise à partir du corps fluorescent (48A, 48B), et d'imager une lumière réfléchie de la pseudo-lumière blanche avec le capteur d'image (52) étant un capteur d'image couleur, afin ainsi d'acquérir les premier aux troisième signaux d'image.

4. Système de diagnostic endoscopique (10) selon la revendication 1, dans lequel le dispositif d'endoscope (14) est apte à filtrer une lumière blanche émise à partir d'une source de lumière blanche (84) par le biais d'un filtre à bande étroite (86), et
à éclairer, dans une première trame, le sujet avec une lumière présentant une plage de longueurs d'onde allant de 460 nm à 480 nm, et à imager une lumière réfléchie de la lumière avec le capteur d'image (52) étant un capteur d'image monochromatique pour acquérir le premier signal d'image, et
à éclairer, dans une deuxième trame, le sujet avec une lumière présentant une plage de longueurs d'onde allant de 540 nm à 580 nm, et à imager une lumière réfléchie de la lumière avec le capteur d'image (52) étant le capteur d'image monochromatique pour acquérir le troisième signal d'image, et
à éclairer, dans une troisième trame, le sujet avec une lumière présentant une plage de longueurs d'onde allant de 590 nm à 700 nm, et à imager une lumière réfléchie de la lumière avec le capteur d'image (52) étant un capteur d'image monochromatique pour acquérir le deuxième signal d'image.

5. Système de diagnostic endoscopique (10) selon la revendication 1, dans lequel le dispositif d'endoscope (14) est apte à filtrer une lumière blanche émise à partir d'une source de lumière blanche (84) du dispositif de source lumineuse (12) par le biais d'un premier et d'un second filtres à bande étroite (94A, 94B), afin d'éclairer le sujet avec une lumière présentant une plage de longueurs d'onde allant de 460 nm à 480 nm, et une lumière présentant une plage de longueurs d'onde allant de 540 nm à 700 nm de manière simultanée, et à imager une lumière réfléchie de ces lumières avec le capteur d'image (52) étant un capteur d'image couleur pour acquérir les premier au troisième signaux d'image.

6. Système de diagnostic endoscopique (10), comprenant :
un dispositif de source lumineuse (12) pour émettre une lumière présentant une plage de longueurs d'onde donnée ;
un dispositif d'endoscope (14) présentant un capteur d'image (52), et pour guider la lumière émise à partir du dispositif de source lumineuse (12), éclairer un sujet avec une lumière d'éclairage, et imager une lumière réfléchie de la lumière d'éclairage avec le capteur d'image (52) afin d'acquérir des signaux d'image ;
une section de calcul de niveau de saturation en oxygène d'une quantité de sang (80) pour calculer une quantité de sang et un niveau de saturation en oxygène de l'hémoglobine du sang du sujet sur la base des signaux d'image acquis, et
un dispositif d'affichage d'image (18) pour afficher une distribution de niveau de saturation en oxygène comme image de pseudo-couleur sur la base d'informations sur le niveau de saturation en oxygène,
**caractérisé en ce que** le dispositif d'endoscope (14) est apte à filtrer une lumière blanche émise à partir d'une source de lumière blanche (84) du dispositif de source lumineuse (12) par le biais d'un filtre à bande étroite (86), et
dans une première trame, à éclairer le sujet avec une lumière présentant une plage de longueurs d'onde allant de 530 nm à 550 nm, et à imager une lumière réfléchie de la lumière avec le capteur d'image (52) étant un capteur d'image monochromatique pour acquérir un premier signal d'image ;
dans une deuxième trame, à éclairer le sujet avec une lumière présentant une plage de longueurs d'onde allant de 555 nm à 565 nm, et à imager une lumière réfléchie de la lumière avec le capteur d'image (52) étant un capteur d'image monochromatique pour acquérir un troisième signal d'image, et
dans une troisième trame, à éclairer le sujet avec une lumière présentant une plage de longueurs d'onde allant de 590 nm à 700 nm, et à imager une lumière réfléchie de la lumière avec le capteur d'image (52) étant un capteur d'image monochromatique pour acquérir un deuxième signal d'image, et
la section de calcul de niveau de saturation en oxygène d'une quantité de sang (80) comprend un stockage de corrélation (78) à l'intérieur duquel est stockée une corrélation, d'une part, entre un premier rapport de signaux entre le premier signal d'image et le troisième signal d'image et un second rapport de signaux entre le deuxième signal d'image et le troisième signal d'image, et d'autre part, entre la quantité de sang et le niveau de saturation en oxygène, la section de calcul de niveau de saturation en oxygène d'une quantité de sang (80) étant apte à calculer le premier et le second rapports de signaux à partir du premier au troisième signaux d'image, et à calculer les informations sur la quantité de sang et le niveau de saturation en oxygène correspondant aux premier et second rapports de signaux calculés à l'aide de la corrélation stockée dans le stockage de corrélation (78).
